# EUROPEAN PATENT APPLICATION

(11) **EP 3 949 852 A1**
(43) Date of publication of application: **09.02.2022**
(21) Application number: 20783519.0
(22) Date of filing: 27.03.2020
(51) Int. Cl.: A61B 5/05, A61B 5/107

(54) **BIOLOGICAL DATA MEASUREMENT DEVICE, BIOLOGICAL DATA MEASUREMENT METHOD, AND PROGRAM**

(30) Priority: 29.03.2019 JP 2019068650
(71) Applicant: Tanita Corporation, Tokyo 174-8630 (JP)
(72) Inventor: TAKAHASHI, Masayoshi, Tokyo 174-8630 (JP)
(74) Representative: Barker Brettell LLP
(86) International application number: PCT/JP2020/014328
(87) International publication number: WO 2020/203893

(57) **Abstract**

A biological data measurement apparatus is provided with: an electrode part provided with an energization electrode and a measurement electrode, the electrode part being fixable to an upper limb of a user, and the energization electrode and the measurement electrode being arranged so as to be separated from each other; biological-information measurement means configured to measure biological information of the user via the electrode part; inclination detection means configured to detect an inclination of the electrode part; and correction means configured to correct a measurement result obtained by the biological-information measurement means in accordance with the inclination of the electrode part.

## Description

### TECHNICAL FIELD

The present invention relates to a biological data measurement apparatus, a biological data measurement method, and a program for measuring a biological data of a user.

### BACKGROUND ART

JP5493198B discloses a body composition analyzer as a biological data measurement apparatus of a type in which a user grasps a grip electrode by a hand. In the body composition analyzer of this type, when the user grasps the grip electrode by the hand, an electrical current is applied from the hand to measure the biological data, such as an impedance, etc., and thereby, body-composition-related values, including a body fat percentage for example, of the user are obtained.

In the above-description, when the user is not grasping the grip electrode suitably by changing a grasping position of the grip electrode for every measurement, for example, the biological data measured via the grip electrode is caused to be changed, and therefore, the body composition of the user cannot be obtained accurately.

Thus, the body composition analyzer disclosed in JP5493198B is configured to determine whether or not the grip electrode is grasped suitably by the user and to measure the biological data of the user only when it is determined that the grip electrode is grasped suitably.

### SUMMARY OF INVENTION

The biological data measured by applying the electrical current from the hand is changed in accordance with changes in a muscle contraction rate, a body water content, or the like. In addition, the muscle contraction rate, the body water content, or the like is caused to be changed unconsciously depending on a posture of the user. Therefore, with the above-described body composition analyzer, there is a problem in that, even in a case in which the user is grasping the grip electrode suitably, it is not possible to obtain the body-composition-related values of the user accurately depending on the posture at the time of the measurement.

An object of the present invention is to provide a biological data measurement apparatus capable of obtaining a body-composition-related value of a user accurately regardless of a posture of the user when the biological data is measured.

According to an aspect of the present invention, the biological data measurement apparatus includes: an electrode part provided with an energization electrode and a measurement electrode, the electrode part being fixable to an upper limb of a user, and the energization electrode and the measurement electrode being arranged so as to be separated from each other; biological-information measurement means configured to measure biological information of the user by using the electrode part; inclination detection means configured to detect an inclination of the electrode part; and correction means configured to correct a measurement result obtained by the biological-information measurement means in accordance with the inclination of the electrode part detected by the inclination detection means.

According to this aspect, because the biological information measured by applying the electrical current from the hand can be corrected in accordance with a posture of the user, it is possible to obtain a body-composition-related value of the user more accurately regardless of the posture of the user.

### BRIEF DESCRIPTION OF DRAWINGS

FIG. 1 is a diagram showing an external appearance of a body composition analyzer to which a biological data measurement apparatus in a first embodiment of the present invention is applied.
FIG. 2A is a diagram showing an external appearance of the hand grip provided on a biological data measurement apparatus in the first embodiment.
FIG. 2B is a diagram showing the external appearance of the hand grip provided on a biological data measurement apparatus in the first embodiment.
FIG. 2C is a diagram showing the external appearance of the hand grip provided on a biological data measurement apparatus in the first embodiment.
FIG. 3A is a diagram for explaining a usage state of the hand grip.
FIG. 3B is a diagram for explaining the usage state of the hand grip.
FIG. 4 is a block diagram showing an example of a functional configuration of a body composition analyzer of the first embodiment.
FIG. 5A is a plan view of an acceleration sensor provided in the hand grip.
FIG. 5B is a side view of the acceleration sensor provided in the hand grip.
FIG. 5C is a diagram for explaining an arrangement example of the acceleration sensor provided in the hand grip.
FIG. 6 is a flowchart showing a processing procedure for a body composition measurement processing in the first embodiment.
FIG. 7 is a diagram for explaining a posture (a normal posture) that is recommended when a bioelectrical impedance of the user is to be measured by using the body composition analyzer of the first embodiment.
FIG. 8A is a diagram for explaining a posture (an abnormal posture) that is inappropriate when the bioelectrical impedance of the user is to be measured by using the body composition analyzer of the first embodiment.
FIG. 8B is a diagram for explaining the posture (the abnormal posture) that is inappropriate when the bioelectrical impedance of the user is to be measured by using the body composition analyzer of the first embodiment.
FIG. 9 is a diagram showing an example of the posture (the abnormal posture) that is inappropriate when the bioelectrical impedance of the user is to be measured by using the body composition analyzer of the first embodiment.
FIG. 10 is a diagram showing an example of the posture (the abnormal posture) that is inappropriate when the bioelectrical impedance of the user is to be measured by using the body composition analyzer of the first embodiment.
FIG. 11A is a diagram for explaining the hand grip in a second embodiment.
FIG. 11B is a diagram for explaining the hand grip in the second embodiment.
FIG. 11C is a diagram for explaining the hand grip in the second embodiment.
FIG. 11D is a diagram for explaining the hand grip in the second embodiment.
FIG. 12 is a block diagram showing an example of the functional configuration of the body composition analyzer of the second embodiment.
FIG. 13 is a flowchart showing the processing procedure for a mode switching processing in the second embodiment.

### DESCRIPTION OF EMBODIMENTS

### <First Embodiment>

In the following, a first embodiment of the present invention will be explained with reference to the attached drawings, etc. In this embodiment, an example in which a biological data measurement apparatus according to the present invention is applied to a body composition analyzer capable of measuring a body information, such as body fat, etc., will be described.

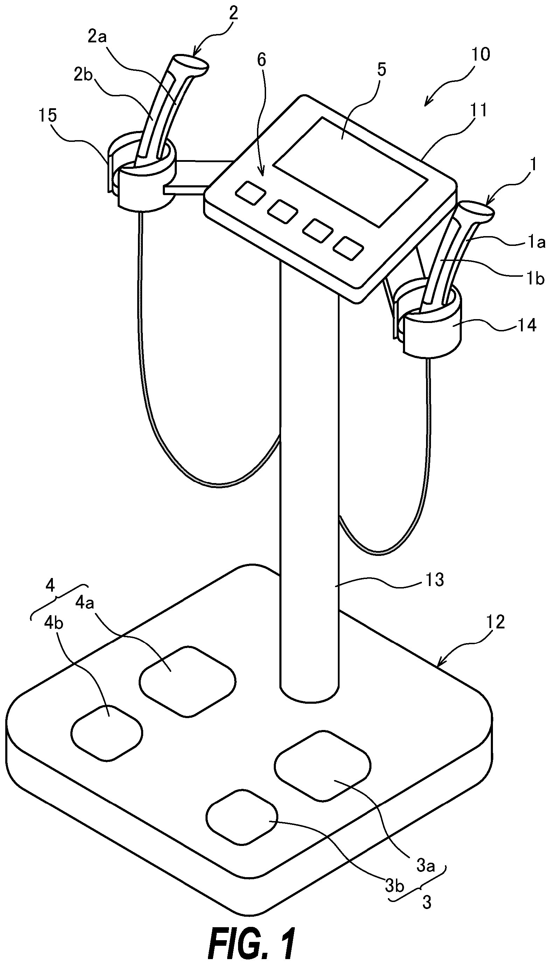

FIG. 1 is a diagram showing an external appearance of a body composition analyzer 10 according to this embodiment. As shown in FIG. 1, the body composition analyzer 10 is provided with hand-side measurement portions (hand grips) 1, 2 and foot-side measurement portions 3, 4 that measure a bioelectrical impedance of a user, a display portion 5, and an operation portion 6. The body composition analyzer 10 is further provided with: a hand-side unit 11 provided with the display portion 5 and the operation portion 6; a foot-side unit 12 provided with the foot-side measurement portions 3, 4; a supporting column 13 that is fixed to the foot-side unit 12 to support the hand-side unit 11; and the holder portions 14, 15 that respectively hold the hand grips 1, 2 in a detachable manner.

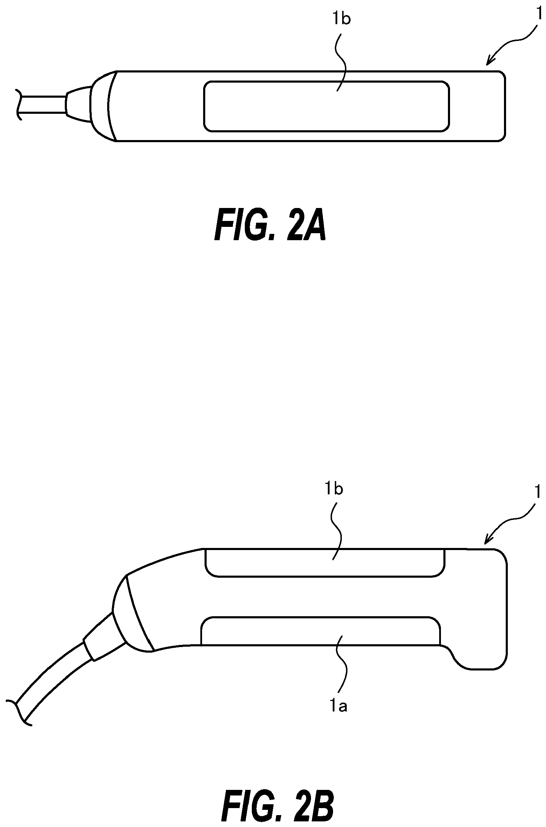

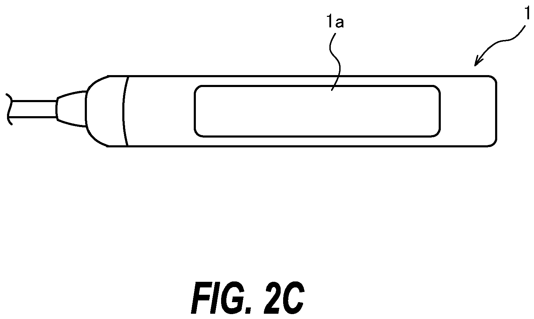

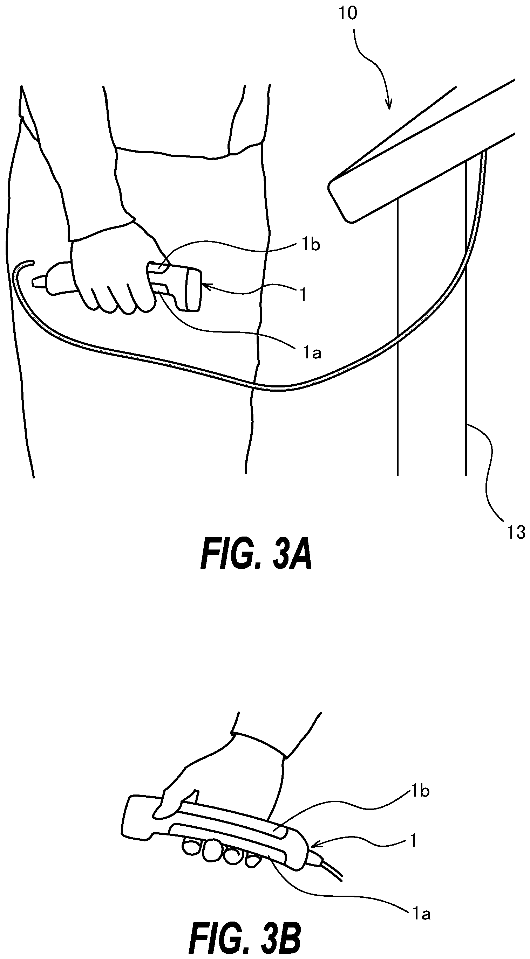

The hand grips (the electrode parts) 1, 2 are each configured so as to measure the bioelectrical impedance of the user, in a state in which it is fixed to an upper limb of the user, specifically in a state in which it is grasped by the hand of the user, by applying the electrical current to the hand and by detecting the voltage. The hand grip 1 is provided with an electrical-current electrode 1a that applies the electrical current to the right hand of the user and a voltage electrode 1b that detects the voltage caused in the right hand by the electrical current applied to the right hand of the user from the electrical-current electrode 1a. The hand grip 2 is provided with an electrical-current electrode 2a that applies the electrical current to the left hand of the user and a voltage electrode 2b that detects the voltage caused in the left hand by the electrical current applied to the left hand of the user from the electrical-current electrode 2a. The hand grips 1, 2 in this embodiment are connected to the hand-side unit 11 via a cable capable of transmitting measurement data. Details of the hand grips 1, 2 will be described later with reference to FIGs. 2, 3, and so forth.

The foot-side measurement portions 3, 4 are each configured so as to measure the bioelectrical impedance of the user by applying the electrical current to the foot and by detecting the voltage. The foot-side measurement portion 3 is provided with an electrical-current electrode 3a that applies the electrical current to the right foot of the user and a voltage electrode 3b that detects the voltage caused in the right foot by the electrical current applied to the right foot of the user from the electrical-current electrode 3a. The foot-side measurement portion 4 is provided with an electrical-current electrode 4a that applies the electrical current to the left foot of the user and a voltage electrode 4b that detects the voltage caused in the left foot by the electrical current applied to the left foot of the user from the electrical-current electrode 4a. The foot-side unit 12 provided with the foot-side measurement portions 3, 4 may also be configured so as to have a function of a scale.

As described above, the body composition analyzer 10 in this embodiment is configured such that the hand-side unit 11 to which the hand grips 1, 2 are connected and the foot-side unit 12 provided with the foot-side measurement portions 3, 4 are integrally formed via the supporting column 13 and so as to be capable of measuring the bioelectrical impedances of a whole body and the respective parts of the user by using eight electrodes that are arranged separately from each other so as to be capable of being respectively brought into contact with both hands and both feet. However, the biological data measurement apparatus according to this embodiment is not necessarily applied to an eight-electrode type body composition analyzer having the eight electrodes. The biological data measurement apparatus according to the present invention may also be applied to a four-electrode type body composition analyzer that measures the bioelectrical impedance of the user by using a total of four electrodes provided on the hand grips 1, 2, without requiring the foot-side measurement portions 3, 4.

The display portion 5 functions as informative means that informs the user of measurement results, etc. In the display portion 5, a liquid crystal display panel such as an LCD (Liquid Crystal Display), etc. is employed for example.

The operation portion 6 functions as an input interface for receiving input operation by the user. For example, the operation portion 6 in this embodiment includes a plurality of operation buttons including input buttons for inputting basic biological information, such as the stature, sex, age, and so forth, a power button for turning ON/OFF the power of the body composition analyzer 10, and so forth. However, in a case in which a touch panel functioning as the input interface is employed for the display portion 5, the operation portion 6 may be omitted, and the function of the operation portion 6 may be achieved by the display portion 5.

In the following, the details of the hand grips 1, 2 in this embodiment will be described. However, the hand grip 1 and the hand grip 2 are formed left-right symmetry in the body composition analyzer 10, and the hand grip 1 and the hand grip 2 have the same basic configuration and function except for a difference in that the measurement object is the right hand or the left hand. Thus, in the following, the details of the hand grip 1 whose measurement object is the right hand of the user will be described as a representative.

FIGs. 2A to 2C and FIGs. 3A and 3B are diagrams for explaining the details of the hand grip 1. FIGs. 2A to 2C are diagrams for explaining an external appearance of the hand grip 1. FIGs. 3A and 3B are diagrams for explaining a usage state of the hand grip 1. FIG. 3A shows a suitable usage state of the hand grip 1, and FIG. 3B shows an example of an inappropriate usage state of the hand grip 1.

The hand grip 1 is used in a state in which it is grasped by the right hand of the user after being removed from the holder portion 14. In addition, as shown in FIG. 3A, when the hand grip 1 is grasped by the user, it is preferable that the hand grip 1 be grasped firmly so as not to be wobbled at least during the measurement by causing the finger tips side of the hand to come into contact with the electrical-current electrode 1a and the palm to come into contact with the voltage electrode 1b. If the hand grip 1 is grasped too loosely as shown in FIG. 3B, a measurement accuracy is substantially deteriorated due to the insufficient contact between the hand of the user and the respective electrodes, wobbling of the hand grip 1 during the measurement, or the like, and so, it is not preferred.

In addition, as shown in FIG. 3A, the hand grip 1 is preferably used in a state in which the hand grip 1 is held by the right hand of the user such that its longitudinal direction is in the horizontal direction with respect to the ground surface. Based on this state, FIG. 2A shows an upper surface of the hand grip 1, FIG. 2B shows a side surface of the hand grip 1, and FIG. 2C shows a lower surface of the hand grip 1. As shown in the figures, in the hand grip 1, the electrical-current electrode 1a serving as an energization electrode and the voltage electrode 1b serving as a measurement electrode are arranged so as to be separated from each other. In addition, the electrical-current electrode 1a is provided on the lower surface of the hand grip 1, and the voltage electrode 1b is provided on the upper surface of the hand grip 1.

Because the hand grip 1 is configured as described above, in a state in which being grasped by the right hand of the user as shown in FIG. 3A, the hand grip 1 can apply the electrical current to the finger tips side of the right hand of the user and suitably detect the voltage from the palm of the right hand of the user. Then, the body composition analyzer 10 can measure (calculate) the bioelectrical impedance of the user on the basis of the electrical current applied by at least a pair of hand grips 1 and 2 and the respective detected values of the voltage, and further, the body composition analyzer 10 can calculate a body-composition-related value of the user such as a body fat percentage, a visceral fat level, and so forth on the basis of the measured value of the bioelectrical impedance that has been measured. Hereinafter, the value of the bioelectrical impedance measured is also referred to as the measured value.

However, the bioelectrical impedance of the user that is measured on the basis of the respective values for the electrical current applied to the user and the voltage detected by using the hand grip 1 is changed depending on the state of the user at the time of the measurement. For example, when the body water content of the user is changed (moved) by the posture of the user, the bioelectrical impedance is also changed in accordance with the amount of change (the amount of movement) of the body water content. In addition, also when the muscle of the user is contracted in accordance with the posture and the cross-sectional area of the muscle is changed, the bioelectrical impedance is changed. In addition, the bioelectrical impedance is also changed in accordance with a change in the shape of the joint of the user, a change in a blood flow, and so forth.

In other words, there is a problem in that when the bioelectrical impedance of the user is measured by using the hand grip 1, the measured value of the bioelectrical impedance is changed due to the posture of the user. Therefore, when the bioelectrical impedance is measured at the posture that is not suitable, the body-composition-related value of the user that is calculated on the basis of the measured value of the bioelectrical impedance also becomes inaccurate. The biological data measurement apparatus of this embodiment has been invented in light of such a problem, and the biological data measurement apparatus is configured so as to be capable of calculating the body-composition-related value of the user as accurately as possible regardless of the posture of the user at the time of the measurement.

Specifically, the body composition analyzer 10 according to the first embodiment is configured so as to detect an inclination (angle) of the hand grip 1 as a parameter indicative of the posture of the user. The hand grip 1 of this embodiment is provided with a three-axis acceleration sensor 7 serving as means for detecting the inclination and detects an angle of each of angles (the X axis, the Y axis, and the Z axis) as the parameter indicative of the posture of the user. The body composition analyzer 10 then corrects the measured value of the bioelectrical impedance measured by the hand grip 1 in accordance with the detected value (the X-axis angle, the Y-axis angle, and the Z-axis angle) obtained by the acceleration sensor 7 provided in the hand grip 1. By doing so, even if the posture of the user at the time of the measurement is disturbed, the body composition analyzer 10 can correct the measured value of the bioelectrical impedance in accordance with the posture, and thereby, the body composition analyzer 10 improve the accuracy of the body-composition-related value calculated on the basis of the measured value of the bioelectrical impedance. As a result, the body composition analyzer 10 in this embodiment can calculate the body-composition-related value of the user accurately regardless of the posture of the user at the time of the measurement. The details of the functions of the body composition analyzer 10 in this embodiment will be described below.

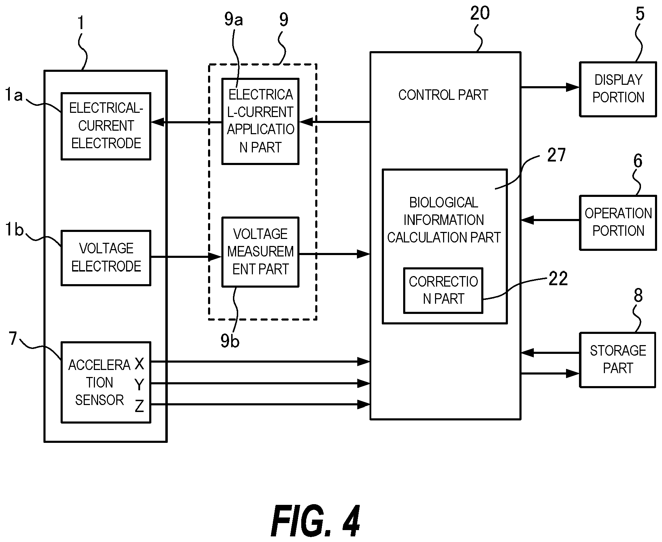

FIG. 4 is a block diagram showing a main functional configuration of the body composition analyzer 10 in this embodiment. As described above, because the hand grip 1 and the hand grip 2 have the same basic configuration and function except for a difference in that it is adapted to the right hand or to the left hand, FIG. 4 only shows the hand grip 1 as a representative. In addition, because the foot-side measurement portions 3, 4 for the foot-side unit 12 are not essential configurations as described above, the description thereof is omitted.

As its functional configuration, the body composition analyzer 10 is mainly provided with: in addition to the display portion 5, the operation portion 6, the hand grip 1, the electrical-current electrode 1a, the voltage electrode 1b, and the acceleration sensor 7, that are described above, a storage part 8; the bioelectrical impedance measurement part 9; and a control part 20 including a biological information calculation part 27 and a biological information the correction part 22.

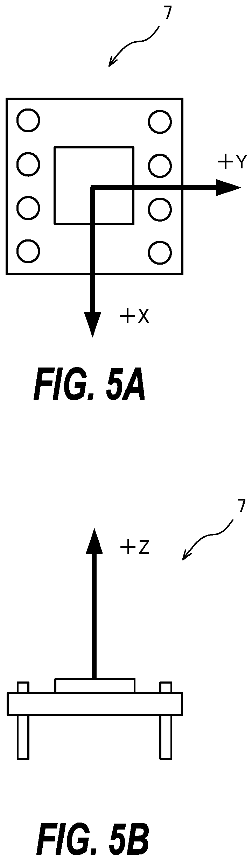

The acceleration sensor 7 serving as inclination detection means is provided in the hand grip 1 and detects the angles (the X-axis angle, the Y-axis angle, and the Z-axis angle) of the hand grip 1. The details of the acceleration sensor 7 will be described with reference to FIGs. 5A to 5C.

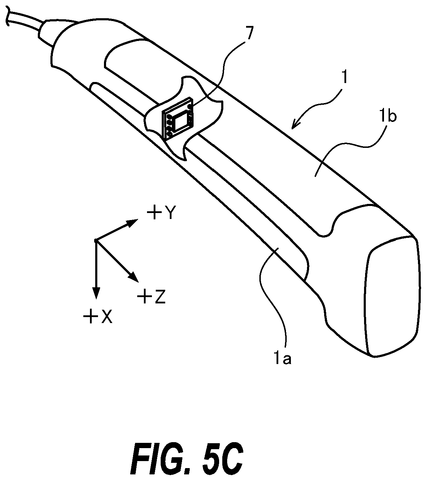

FIGs. 5A to 5C are diagrams for explaining the acceleration sensor 7 provided in the hand grip 1. FIGs. 5A and 5B show the axial directions detected by the acceleration sensor 7, and FIG. 5C shows an arrangement example of the acceleration sensor 7 in the hand grip 1. As shown in the plan view in FIG. 5A for example, the acceleration sensor 7 in this embodiment detects a predetermined one direction that is in parallel with the plane direction of the acceleration sensor 7 as the X axis, and similarly, detects the direction that is in parallel with the plane direction and that is perpendicular to the X axis as the Y axis. Furthermore, as shown in the side view in FIG. 5B, the acceleration sensor 7 detects the vertical direction relative to the plane direction as the Z axis.

As shown in FIG. 5C, the acceleration sensor 7 is arranged in the hand grip 1, which is in a state in which the longitudinal direction thereof is inclined so as to be in parallel with the ground surface, such that the longitudinal direction of the hand grip 1 coincides with the Z axis direction, the lateral direction that is perpendicular to the longitudinal direction of the upper surface of the hand grip 1 (see FIG. 2B) coincides with the Y axis direction, and the X axis direction coincides with the vertical direction relative to the ground surface. In the above, the position of the acceleration sensor 7 in the longitudinal direction of the hand grip 1 may be set appropriately by taking a detection sensitivity into consideration. As described above, by providing the acceleration sensor 7 in the hand grip 1, it is possible to detect the angles (the X-axis angle, the Y-axis angle, and the Z-axis angle) of the hand grip 1 that is grasped by the user. Although not illustrated on the figures, the acceleration sensor 7 is also arranged in the hand grip 2 in a similar manner. The description will be continued below by referring back to FIG. 4.

The bioelectrical impedance measurement part 9 is configured of an electrical-current application part 9a that is electrically connected to the electrical-current electrode 1a and a voltage measurement part 9b that is electrically connected to the voltage electrode 1b. The electrical-current application part 9a applies the AC electrical current to the finger tips of the hand of the user via the electrical-current electrode 1a, and the voltage measurement part 9b detects the voltage in the palm of the user via the voltage electrode 1b.

The biological information calculation part 27 functions as biological-information measurement means for measuring the biological information of the user by using the hand grips 1 and 2. The biological information in this description includes the bioelectrical impedance or the body composition. The description will be continued below by assuming that the biological information in this embodiment is the bioelectrical impedance. The biological information calculation part 27 of this embodiment calculates, in the bioelectrical impedance measurement part 9, the bioelectrical impedance of the user on the basis of the respective values for the supplied electrical current and the detected voltage. A so called BIA (Bioelectrical Impedance Analysis) may be used as a method for calculating the bioelectrical impedance, and the method may be similar to those for a known body composition analyzer except that the correction, which will be described below, will be performed.

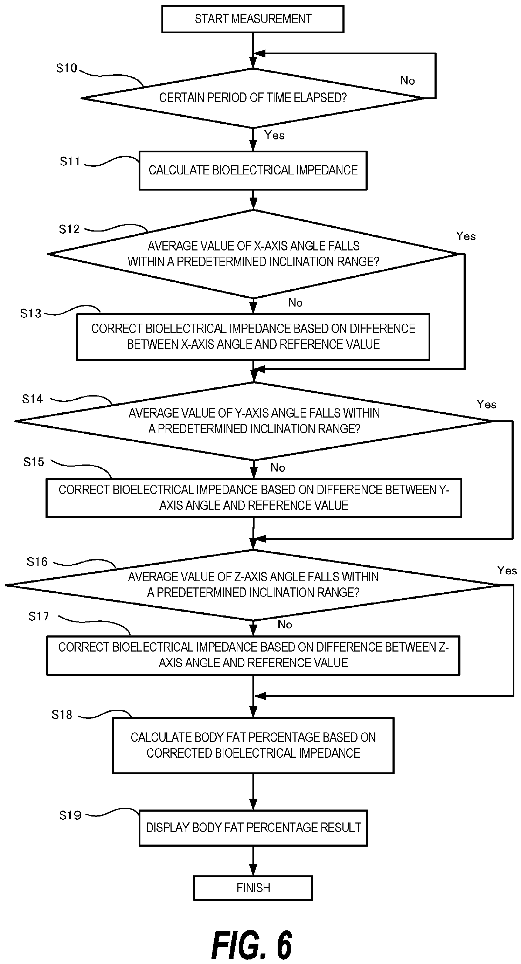

The biological information correction part 22 (hereinafter, simply referred to as the correction part 22) corrects the measured value of the bioelectrical impedance of the user calculated by the biological information calculation part 27 on the basis of the angles (the X-axis angle, the Y-axis angle, and the Z-axis angle) of the hand grip 1 detected by the acceleration sensor 7. The details of the correction will be described below with reference to FIG. 6.

A control program for controlling an operation of the body composition analyzer 10 is stored in the storage part 8. In other words, the storage part 8 functions as a computer readable storage medium in which the program that realizes the functions of the biological data measurement apparatus of this embodiment is recorded. The storage part 8 is formed of a nonvolatile memory (ROM; Read Only Memory), a volatile memory (RAM; Random Access Memory), and so forth.

In addition, the storage part 8 also stores a map data, etc. that is referred to during the correction of basic biological information input to the operation portion 6, the corrected bioelectrical impedance information, and the measured value of the bioelectrical impedance according to the X-axis angle, the Y-axis angle, or the Z-axis angle.

The control part 20 is configured of a central processing unit (CPU), an input-output interface connected to the above-described respective functional components, and a bus for mutually connecting these components. The control part 20 controls respective parts of the body composition analyzer 10 via the input-output interface by reading out the control program stored in the storage part 8 and causing the central processing unit to execute the control program.

More specifically, the control part 20 controls each of the hand grips 1 and 2, the display portion 5, the operation portion 6, the bioelectrical impedance measurement part 9, the storage part 8, the biological information calculation part 27, and the correction part 22 and executes various arithmetic processing required for controlling these components. In addition, in order to realize the respective functions, which will be described below, the control part 20 has functions as: body composition calculation means that calculates the body composition of the user (the body fat percentage in this embodiment) on the basis of the measured value of the bioelectrical impedance; abnormal posture determination means that determines whether or not the inclinations of the hand grips 1, 2 that have detected fall within a predetermined inclination range; and abnormal-posture-degree calculation means that calculates a difference between the inclinations of the hand grips 1, 2 that are expected when the recommended posture of the user is maintained and the inclinations of the hand grips 1, 2 that have been detected.

Subsequently, the details of the processing (a body composition measurement processing) for measuring the body composition of the user executed by the body composition analyzer 10 will be described. In this example, an example in which a body fat percentage is calculated as a kind of the body composition of the user will be shown. The body composition measurement processing described below is realized based on the control program stored in the storage part 8.

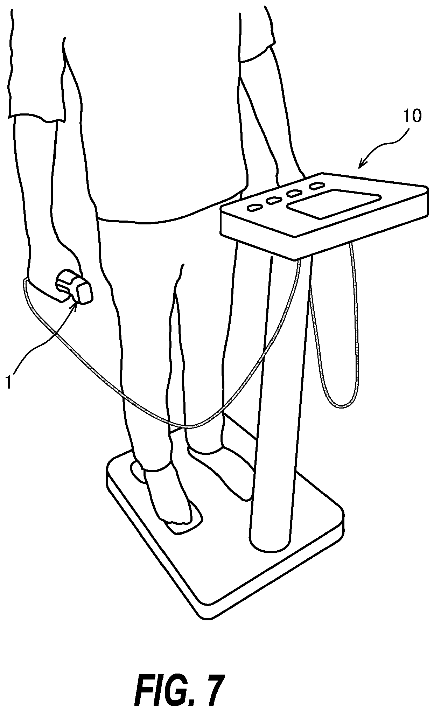

In the above-description, before explaining the flowchart, a situation when the body composition of the user is measured by the user by using the body composition analyzer 10 will be explained. The user who wants to measure the body fat percentage of himself/herself first removes the hand grips 1, 2 from the holder portions 14, 15, respectively, and grasps the hand grip 1 by the right hand and the hand grip 2 by the left hand (see FIG. 3). Then, the user maintains the posture shown in FIG. 7 while the bioelectrical impedance is measured via the hand grips 1, 2. This requirement of maintaining such a posture is informed to the user by, for example, a description in an instruction, in advance, or via the display portion 5 just before the start of the measurement. The posture (the normal posture) shown in FIG. 7 is the posture recommended for suitably measuring the bioelectrical impedance of the user by the body composition analyzer 10 and is the posture serving as a reference for determining whether or not the correction, which will be described below, is required for the measured value of the bioelectrical impedance.

In the normal posture, as shown in the figure, both arms are naturally hanging down from the shoulders in the gravitational direction and are not opened unnaturally in front-back/left-right directions, and in addition, the wrists are not bent. In such a posture, especially the body water content in the upper body do not change abnormally, and the contraction rate, etc. of the muscle of the joints of the wrists or the arms do not change unnaturally, and therefore, it is possible to suitably measure the true bioelectrical impedance of the user. In the body composition analyzer 10 in this embodiment, the X-axis angle, the Y-axis angle, and the Z-axis angle to be detected by the acceleration sensor 7 when the user is in such a normal posture are estimated in advance from experimentally obtained measured values, etc., and the estimated values are stored in advance in the storage part 8 as reference values for determination of the posture, which will be described below. The reference values may be equally set regardless of the physique, an amount of the muscle, or the like of the user, or the reference values may be adjusted (increased/decreased) in accordance with the physique, etc. of the user estimated based on the basic biological information, etc., which has been input to the operation portion 6.

Based on the above description, flows of the body composition measurement processing executed by the body composition analyzer 10 will be described below by following the flowchart.

In step S10, the control part 20 determines whether or not a certain period of time has elapsed without any movement of the user after the measurement of the bioelectrical impedance of the user was started by applying the electrical current to the hand of the user via the electrical-current application part 9a. The certain period of time is a period of time required for suitably measuring the bioelectrical impedance and is, for example, ten seconds. During this period, the user tries not to move in a relaxed state as much as possible. Then, in step S10, the control part 20 acquires the data (the X-axis angle, the Y-axis angle, and the Z-axis angle) related to the inclination of the hand grip 1 from the acceleration sensor 7. In other words, the processing in step S10 functions as an inclination detection step for detecting the inclination of the hand grip 1 as the parameter indicative of the posture of the user. In the above-description, when it is determined that the user has moved before the certain period of time is elapsed by, for example, detecting the change in the detected values from the voltage electrode 1b, 2b and/or the detected value from the acceleration sensor 7, the control part 20 re-executes the processing in step S10 from the beginning. In a case in which the control part 20 re-executes the processing in step S10, the user may be informed via the display portion 5 that he/she needs to adjust his/her posture to a more suitable posture. When it is determined that the detected values from the voltage electrode 1b, 2b and the detected value from the acceleration sensor 7 do not change abnormally and that the certain period of time has moved without the movement of the user, the processing in step S11 is executed.

In step S11, the biological information calculation part 27 calculates the bioelectrical impedance of the user by known methods on the basis of the basic biological information of the user and of the electrical current applied from the electrical-current electrodes 1a, 2a and the voltage detected by the voltage electrode 1b, 2b in step S10. When the bioelectrical impedance is calculated, the processing in step S12 is executed.

In step S12, the control part 20 determines whether or not the average value of the X-axis angle over the certain period of time required for measuring the bioelectrical impedance in step S10 falls within the predetermined inclination range. The predetermined inclination range in the above description is set on the basis of the detected value of the X-axis angle (the reference value) that is expected when the user is in the normal posture. For example, in this embodiment, a range of ±5° of the reference value is set as the predetermined inclination range of the X-axis angle by considering a measurement error that can be allowed from the viewpoint of ensuring the accuracy of the measurement result of the body fat percentage. For example, when the reference value is set as 0°, if the detected X-axis angle falls within the range of ±5°, in other words, if the average value of the X-axis angle falls within the range of ±5° with respect to the reference value, the posture is determined as being the normal posture, and if the average value falls outside the range of ±5°, the posture is determined as being the abnormal posture.

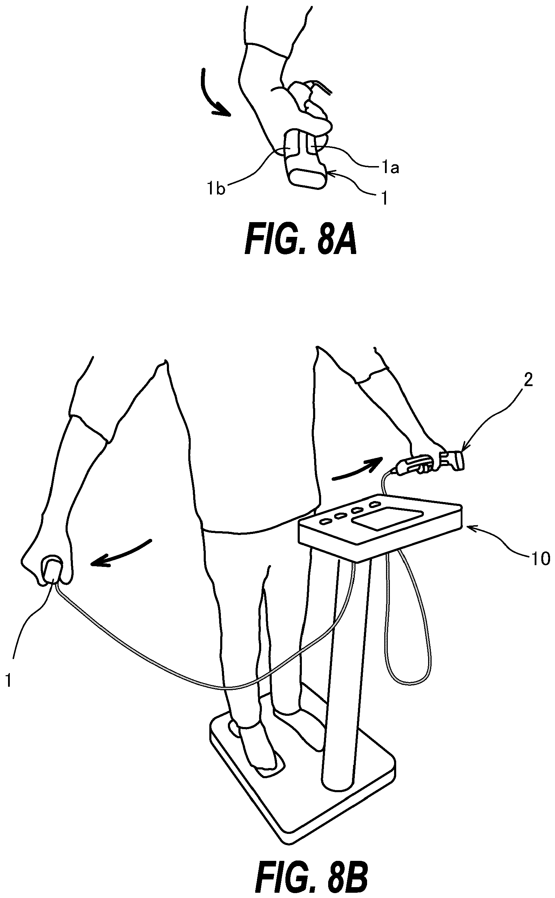

FIGs. 8A and B are diagrams for explaining an example of the posture (the abnormal posture) with which it is determined that the X-axis angle does not fall within the predetermined inclination range in step S12.

FIG. 8A shows a diagram for explaining the example in which the posture of the user is determined as being the abnormal posture due to a degree of the curvature of the wrist. As shown in the figure, the user in this example is unnaturally bending the wrist so as to roll the hand grip 1 inwards (towards the body) while grasping the hand grip 1. In such a posture, especially the shape of the joint of the right the wrist is different from that in the normal posture, and the muscle contraction rate, etc. of the arm (especially, the forearm) is changed relative to the case in which the user is in the normal posture, and therefore, the bioelectrical impedance to be measured is changed.

When the user is in such an abnormal posture, with the body composition analyzer 10 in this embodiment, for example, the X-axis angle detected by the acceleration sensor 7 at the time of the measurement is shifted inwards (towards the body) with respect to the reference value of the X-axis angle (for example 0°) that is set in the vertical direction relative to the ground surface. Therefore, the control part 20 can acquire a difference (the X-axis angle difference θ1) between the X-axis angle detected via the acceleration sensor 7 provided in the hand grip 1 and the reference value serving as the parameter indicative of the degree of the curvature of the wrist.

In addition, FIG. 8B shows a diagram for explaining the example in which the posture of the user is determined as being the abnormal posture due to a degree of opening of the arm. As shown in the figure, the user in this example is unnaturally opening the arms outwardly (towards the side away from the body) while grasping the hand grip 1. When the user is in such a posture, the body water content of the user is changed (moved) from the state in the normal posture, and therefore, the bioelectrical impedance to be measured is changed in accordance with the amount of change (the amount of movement) of the body water content. In addition, the contraction rate of the muscle around the shoulder or the muscle of the chest (for example, a deltoid muscle or a pectoral muscle) is also changed relative to the case in which the user is in the normal posture, and therefore, these are also factors for the change in the bioelectrical impedance.

When the user is in such an abnormal posture, for example, according to the body composition analyzer 10 in this embodiment, the X-axis angle detected by the acceleration sensor 7 at the time of the measurement is shifted outwards (towards the side away from the body) with respect to the reference value (for example 0°) of the X-axis angle that is set in the vertical direction relative to the ground surface. Therefore, the control part 20 can acquire a difference (the X-axis angle difference θ2) between the X-axis angle detected via the acceleration sensor 7 provided in the hand grip 1 and the reference value as the parameter indicative of the degree of opening of the arm.

The control part 20 determines whether or not the difference (for example, the X-axis angle difference θ1 or θ2) between the average value of the X-axis angle thus detected and the reference value falls within a predetermined range, in other words, determines whether or not the average value of the X-axis angle falls within the predetermined inclination range, and when the average value of the X-axis angle falls within the predetermined inclination range, the control part 20 determines that the posture of the user is the normal posture and executes the processing in step S14. When it is determined that the average value of the X-axis angle does not fall within the predetermined inclination range, the control part 20 determines that the posture of the user is the abnormal posture and executes the processing in step S13.

In step S13, the correction part 22 corrects the measured value of the bioelectrical impedance, which has been calculated in step S11, on the basis of the difference between the X-axis angle detected via the acceleration sensor 7 and the reference value. It is preferable that a correction level be adjusted in accordance with the difference (the X-axis angle difference θ1 or θ2) between the detected X-axis angle and the reference value. For example, the correction is performed such that the larger the X-axis angle difference θ2 is, the smaller the value of the bioelectrical impedance becomes. The correction level of the bioelectrical impedance based on the X-axis angle differences θ1, θ2 is adjusted appropriately such that the bioelectrical impedance after correction takes a suitable value. For example, the map data, in which the correction levels suitable for experimentally derived X-axis angle differences θ1, θ2 are set, is stored in advance, and the correction level is decided as the correction part 22 refers to the map data. In addition, the correction level based on the X-axis angle differences θ1, θ2 may be equally set regardless of the physique, an amount of the muscle, or the like of the user or may be adjusted (increased/decreased) in accordance with the physique, etc. of the user estimated based on the basic biological information, etc. input to the operation portion 6.

In addition, the correction level based on the X-axis angle differences θ1, θ2 may be adjusted in accordance with a mode of the abnormal posture of the user. For example, in the posture of the user, the degree of opening of the arm has a greater effect on the change in the bioelectrical impedance to be measured than the degree of the curvature of the wrist. Therefore, for example, weighting may be performed such that, even if the absolute values of the X-axis angle differences θ1, θ2 are the same, the X-axis angle difference θ2 indicative of the degree of opening of the arm has a greater influence on the correction level than the X-axis angle difference θ1 indicative of the degree of the curvature of the wrist.

In the above description, it is possible to determine the mode of the abnormal posture of the user on the basis of, for example, the shifted direction (for example, positive and negative) of the X-axis angle detected by the acceleration sensor 7 assuming that the X-axis angle shows the reference value when the user is in the normal posture. In addition, in order to grasp the mode of the abnormal posture of the user more accurately, the hand grip 1 may be configured so as to be capable of discriminating the mode by not only using the acceleration sensor 7, but also, by combining, for example, the detected values from other sensors, such as a gyro sensor, a distance sensor, or the like, or alternatively, by using an image recognition, etc. On the other hand, the correction level may be equally set in accordance with the absolute values of the X-axis angle differences θ1, θ2 without considering the mode of the abnormal posture. When the measured value of the bioelectrical impedance is corrected on the basis of the X-axis angle differences θ1, θ2, the processing in step S14 is executed subsequently.

In step S14, the control part 20 determines whether or not the average value of the Y-axis angle over the certain period of time required for measuring the bioelectrical impedance in step S10 falls within the predetermined inclination range. Similarly to the X-axis angle, the predetermined inclination range in this description is set on the basis of the detected value of the Y-axis angle (the reference value) that is expected when the user is in the normal posture.

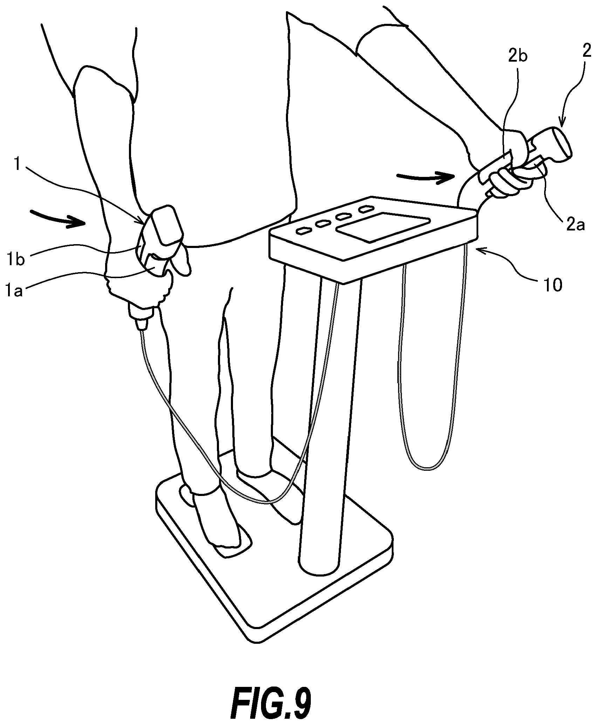

FIG. 9 is a diagram for explaining an example of the posture (the abnormal posture) with which it is determined that a difference between the average value of the Y-axis angle and the reference value (the Y-axis angle difference) does not fall within a predetermined range in step S14.

FIG. 9 shows a diagram for explaining an example in which the posture is determined as being the abnormal posture due to the angle of the arm in the front-back direction. As shown in the figure, the user in this example is excessively lifting the arm grasping the hand grip 1 towards the front. With such a posture, the body water content, the contraction rate of the muscle around the shoulder, or the like of the user is changed relative to the case in which the user is in the normal posture, and therefore, the bioelectrical impedance to be measured is caused to be changed. Although not illustrated, the same is applied to the case in which the arm is excessively lowered towards back.

When the user is in such an abnormal posture, according to the body composition analyzer 10 in this embodiment for example, the Y axis direction detected by the acceleration sensor 7 at the time of the measurement is changed upwards (downwards when the arm is lowered towards the back) relative to the reference value of the Y-axis angle that is set for the parallel direction with the lateral direction of the hand grip 1. Therefore, the control part 20 can acquire, as the parameter indicative of a degree of the lifting of the arm towards the front, the difference between the Y-axis angle detected via the acceleration sensor 7 provided in the hand grip 1 and the reference value, in other words, the Y-axis angle difference .

The control part 20 determines whether or not the difference between the average value of the Y-axis angle detected as described above and the reference value falls within the predetermined range, in other words, whether or not the average value of the Y-axis angle falls within the predetermined inclination range, and when the average value of the Y-axis angle falls within the predetermined inclination range, the control part 20 determines that the posture of the user is the normal posture and executes the processing in step S16. When it is determined that the average value of the Y-axis angle does not fall within the predetermined inclination range, the control part 20 determines that the posture of the user is the abnormal posture and executes the processing in step S15.

In step S15, the correction part 22 corrects the bioelectrical impedance, which has been calculated in step S11, on the basis of the difference between the Y-axis angle detected via the acceleration sensor 7 and the reference value (the Y-axis angle difference), or if required, the correction part 22 further corrects the bioelectrical impedance, which has been corrected in step S13. Similarly to the case described above for the X-axis angle differences θ1, θ2, it is preferable that the correction level be adjusted in accordance with the difference between the Y-axis angle detected and the reference value (the Y-axis angle difference). When the bioelectrical impedance is corrected on the basis of the Y-axis angle difference, the processing in step S16 is executed subsequently.

In step S16, the control part 20 determines whether or not the average value of the Z-axis angle over the certain period of time required for measuring the bioelectrical impedance in step S10 falls within the predetermined inclination range. Similarly to the X-axis angle and the Y-axis angle, the predetermined inclination range in the above description is set on the basis of the detected value of the Z-axis angle (the reference value) that is expected when the user is in the normal posture.

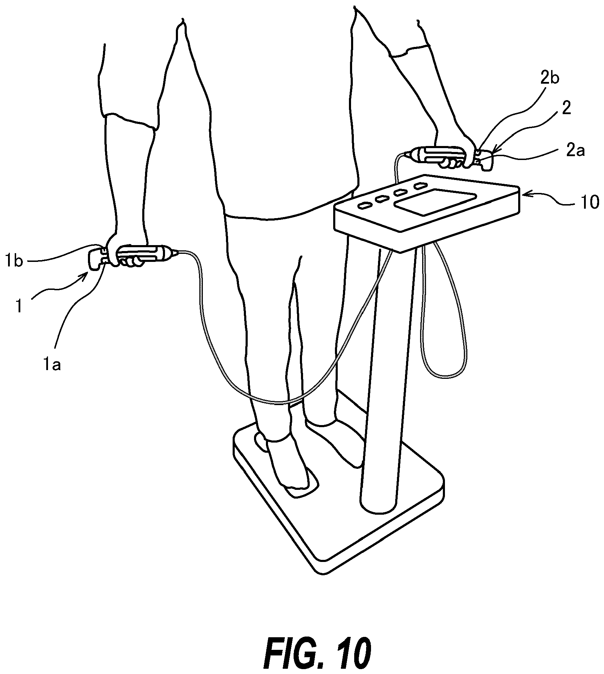

FIG. 10 is a diagram for explaining an example of the posture (the abnormal posture) with which it is determined that a difference between the average value of the Z-axis angle and the reference value (the Z-axis angle difference) does not fall within a predetermined range in step S16.

FIG. 10 shows a diagram for explaining the example in which the posture of the user is determined as being the abnormal posture due to the degree of twisting of the arm. As shown in the figure, the user in this example is twisting the arm grasping the hand grip 1 in the clockwise direction (the left hand is twisted in the anti-clockwise direction) excessively. With such a posture, the contraction rate, blood flow, or the like of the muscle of the arm of the user is changed relative to the case in which the user is in the normal posture, and therefore, the bioelectrical impedance to be measured is changed.

When the user is in the abnormal posture as described above, according to the body composition analyzer 10 in this embodiment for example, the Z axis direction detected by the acceleration sensor 7 at the time of the measurement is changed relative to the reference value of the Z axis direction that is set for the parallel direction with the lateral direction of the hand grip 1. Therefore, the control part 20 can acquire the difference between the Z-axis angle detected via the acceleration sensor 7 provided in the hand grip 1 and the reference value (the Z-axis angle difference) as a parameter indicative of the degree of twisting of the arm.

The control part 20 determines whether or not the difference between the average value of the Z-axis angle detected as described above and the reference value falls within the predetermined range, in other words, whether or not the average value of the Z-axis angle falls within the predetermined inclination range, and when the average value of the Z-axis angle difference falls within the predetermined inclination range, the control part 20 determines that the posture of the user is the normal posture and executes the processing in step S18. When it is determined that the average value of the Z-axis angle does not fall within the predetermined inclination range, the control part 20 determines that the posture of the user is the abnormal posture and executes the processing in step S17.

In step S17, the correction part 22 corrects the bioelectrical impedance, which has been calculated in step S11, on the basis of a difference between the Z-axis angle detected via the acceleration sensor 7 and the reference value (Z-angle difference), or if required, the correction part 22 further corrects the measured value of the bioelectrical impedance that has been corrected in step S13 and step S15. Similarly to the case described above for the X-axis angle differences θ1, θ2, it is preferable that the correction level be adjusted in accordance with the value of the difference between the Z-axis angle detected and the reference value (the Z-axis angle difference). When the bioelectrical impedance is corrected on the basis of the Z-axis angle difference, the processing in step S18 is executed subsequently.

In step S18, the control part 20 calculates the body fat percentage of the user by known methods on the basis of the measured value of the bioelectrical impedance corrected by the above-described flow. When the body fat percentage of the user is obtained suitably, the processing in step S19 is executed subsequently.

In step S19, the control part 20 informs the user of the measurement result for the body fat percentage of the user obtained in step S19 by displaying it on the display portion 5. By doing so, even when the posture of the user at the time of the measurement is abnormal relative to the normal posture, the body composition analyzer 10 can calculate the body fat percentage of the user more accurately compared with the related art regardless of the posture of the user. When the body fat percentage thus calculated is informed to the user, the control part 20 terminates the processing for measuring the body composition of the user.

The above describes the example of the control executed for measuring the body composition of the user by the body composition analyzer 10 in this embodiment. For the processing in steps S10 to step S19 according to the above-described flow, not all of the steps need to be executed in the above-described order. For example, the correction part 22 need not necessarily correct the bioelectrical impedance on the basis of all of the X-axis angle differences θ1, θ2, the Y-axis angle difference, and the Z-axis angle difference, and the correction may be performed on the basis of at least one difference, for example, the X-axis angle difference θ1, θ2. In addition, when the correction part 22 corrects the bioelectrical impedance on the basis of at least two of the X-axis angle differences θ1, θ2, the Y-axis angle difference, and the Z-axis angle difference, the weighting may be performed so as to increase/decrease the correction level by considering a degree of influence of the angle difference for the respective axes on the bioelectrical impedance. In the above, when the correction part 22 performs the correction on the basis of only the X-axis angle differences θ1, θ2, for example, it is not necessarily to employ the three-axis acceleration sensor as the acceleration sensor 7, and a uni-axial acceleration sensor adapted to the X axis may be employed.

In addition, the object to be corrected by the correction part 22 on the basis of the angle differences for the respective axes need not be the bioelectrical impedance. The body composition analyzer 10 may correct the body fat percentage as the body-composition-related value on the basis of at least one of the X-axis angle differences θ1, θ2, the Y-axis angle difference, and the Z-axis angle difference. In other words, the correction part 22 may correct the body fat percentage as the body-composition-related value on the basis of the angles of the hand grip 1 (the X-axis angle, the Y-axis angle, and the Z-axis angle) detected by the acceleration sensor 7. In this case, in the body composition measurement processing, steps S13, S15, and S17 are deleted, and the processing in step S18 is changed to a processing in which the body fat percentage of the user is calculated on the basis of the bioelectrical impedance, which is not calculated in step S11. Then, a processing of correcting the body fat percentage on the basis of at least one of the X-axis angle differences θ1, θ2, the Y-axis angle difference, and the Z-axis angle difference (an inclination angle) detected in steps S12, S14, and S16 may be added between step S18 and step S19. In the above description, in this case, the biological information calculation part 27 functioning as the above-described biological-information measurement means is a function part including the body composition calculation means for calculating the body composition of the user (the body fat percentage in this embodiment) and is configured so as to measure the body composition of the user by using the hand grips 1 and 2.

Next, operational advantages of this embodiment will be described.

According to this embodiment, the biological data measurement apparatus (the body composition analyzer 10) is provided with: the electrode part (the hand grip 1, 2) provided with the energization electrode (the electrical-current electrode 1a, 1b) and the measurement electrode (the voltage electrode 1b, 2b), the electrode part (the hand grip 1, 2) being fixable to the upper limb of the user, and the energization electrode (the electrical-current electrode 1a, 1b) and the measurement electrode (the voltage electrode 1b, 2b) being arranged so as to be separated from each other; the biological-information measurement means (the biological information calculation part 27) configured to measure the biological information of the user by using the hand grip 1, 2; the inclination detection means (the acceleration sensor 7) configured to detect the inclination of the hand grip 1, 2; and the correction means (the biological information correction part 22) configured to correct the measurement result obtained by the biological information calculation part 27 in accordance with the inclination of the hand grip 1, 2 detected by the inclination detection means. With such a configuration, even if the posture of the user at the time of the measurement is the abnormal posture, the body composition analyzer 10 can correct the biological information in accordance with the posture, and therefore, it is possible to accurately measure the biological information of the user regardless of the posture of the user.

In addition, according to this embodiment, the measurement result obtained by the biological information calculation part 27 includes the bioelectrical impedance of the user or the body composition of the user. In addition, the biological data measurement apparatus (the body composition analyzer 10) is further provided with the body composition calculation means (the control part 20) configured to calculate, when the measurement result is the bioelectrical impedance of the user, the body composition of the user on the basis of the bioelectrical impedance corrected by the correction means. With such a configuration, when the measurement result obtained by the biological information calculation part 27 is the body composition, even if the posture of the user at the time of the measurement is the abnormal posture, it is possible to accurately measure the body composition of the user by correcting the body composition of the user in accordance with the posture regardless of the posture of the user.

In addition, when the measurement result obtained by the biological information calculation part 27 is the bioelectrical impedance, even if the posture of the user at the time of the measurement is the abnormal posture, the correction part 22 can correct the bioelectrical impedance in accordance with the posture. Thus, the body composition analyzer 10 can improve the accuracy of the body-composition-related value that is calculated on the basis of the bioelectrical impedance. In the above-description, when an object of the correction is the body composition as described above, the correction level is changed in accordance with the different type of the body composition, for example, the body fat percentage or the amount of the muscle. In contrast, when the measurement result obtained by the biological information calculation part 27 is the bioelectrical impedance, regardless of the type the body composition, the object of the correction can be narrowed to the bioelectrical impedance only, and therefore, a computational load is reduced compared with a case in which each of the specific types is individually corrected in accordance with the specific type of the body composition.

In addition, according to the body composition analyzer 10, the inclination detection means is the acceleration sensor 7 provided in the hand grip 1, 2. With such a configuration, the body composition analyzer 10 can detect the angle of the hand grip 1 as the parameter corresponding to the posture of the user at the time of the measurement.

In addition, the body composition analyzer 10 is further provided with the abnormal posture determination means (the control part 20) configured to determine whether or not the inclination of the hand grip 1, 2 detected falls within the predetermined inclination range. When it is determined that the inclination of the hand grip 1, 2 falls within the predetermined inclination range by the control part 20, the biological information correction part 22 corrects the measured value of the bioelectrical impedance. By doing so, it is possible to more suitably correct the bioelectrical impedance thus measured by considering a slight movement (a shaking), a measurement error, and so forth of the posture of the user at the time of the measurement that can be allowed from the viewpoint of ensuring the accuracy of the measurement result.

In addition, according to the body composition analyzer 10, the predetermined inclination range is set on the basis of the inclination of the hand grip 1, 2 at the time when the user, to which the hand grips 1, 2 are fixed, are holding the recommended posture for measuring the bioelectrical impedance. By doing so, a suitable comparison target for determining the abnormal posture is set, and so, the control part 20 can suitably detect whether or not the posture of the user is abnormal.

In addition, with the body composition analyzer 10, when the user holding the hand grip 1, 2 is measuring the bioelectrical impedance by using the abnormal-posture-degree calculation means (the control part 20), the difference between the inclination of the hand grip 1, 2 at the time when the recommended posture is maintained and the detected inclination of the hand grip 1, 2 is calculated, and the correction is performed such that the larger the difference thus calculated is, the smaller the bioelectrical impedance further becomes. By doing so, the correction level can be adjusted in accordance with the posture of the user (especially, the shift from the recommended posture), and therefore, it is possible to more suitably correct the bioelectrical impedance thus measured and to further improve the accuracy of the body-composition-related value calculated on the basis of the bioelectrical impedance.

### <Second Embodiment>

In the following, the body composition analyzer 10 of a second embodiment to which the biological data measurement apparatus according to the present invention is applied will be described.

Four-electrode or eight-electrode type body composition analyzers (body fat analyzers) that have at least two hand grips and that apply the electrical current to both arms or to the upper and lower limbs are known. In addition, there are known four-electrode type body composition analyzers that are capable of measuring a skinfold thickness of the user by pressing a single electrode part including a pair of voltage electrodes and a pair of electrical-current electrodes against the abdomen, etc. Although these body composition analyzers for different measurement points are configured by using similar electrodes, they are provided as separate products because the number and arrangements of the required electrodes are different.

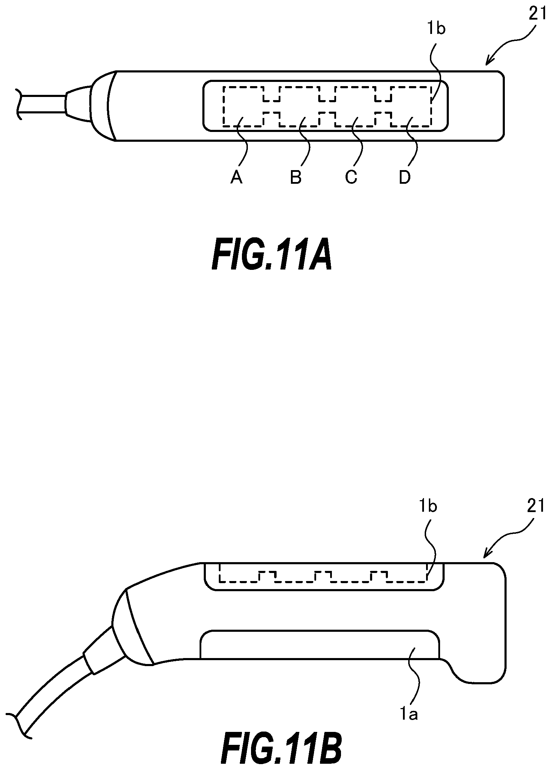

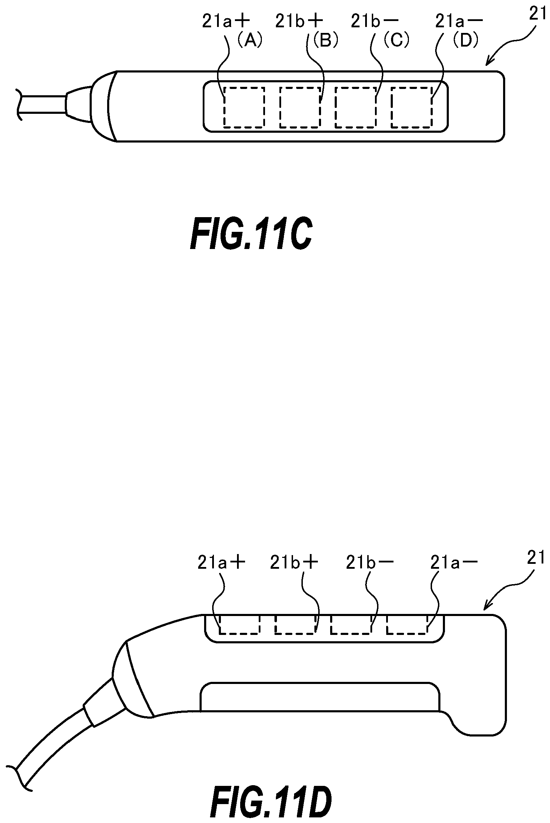

The body composition analyzer 10 in this embodiment has been invented in light of the circumstances described above, and is characterized in that the functions of two types of body composition analyzers with different modes are achieved by a single body composition analyzer. More specifically, a hand grip 21 provided in the body composition analyzer 10 in this embodiment is characterized in that it is configured so as to be capable of being switched between "a normal mode" and "a sebum caliper mode" in the four-electrode or eight-electrode type body composition analyzer (the body fat analyzer). In the normal mode, the hand grip 21 functions as a dual-electrodes body composition analyzer that applies the electrical current to one of the arms (the right arm in this embodiment), and in the sebum caliper mode, the hand grip 21 functions as the four-electrode type body composition analyzer that locally measures the body composition of the user. In the following, the details of the body composition analyzer 10 in this embodiment will be described by using FIGs. 11 to 13. Descriptions of the functions and components that are similar to those in the first embodiment will be omitted.

FIGs. 11A to 11D are diagrams for explaining the hand grip 21 of this embodiment. In the hand grip 21 in this embodiment, a function of realizing "the sebum caliper mode" and a function of switching between "the sebum caliper mode" and "the normal mode" are added to the hand grip 1 described in the first embodiment. These functions can be added not only to the hand grip 1, but also to both of the hand grips 1 and 2. It is also possible to add these functions to only the hand grip 2. Based on the above description, in the following, the hand grip 21 corresponding to the hand grip 1 will be described mainly on differences with the hand grip 1.

FIGs. 11A and 11B are diagrams for explaining the configuration of the hand grip 21 at the time of the normal mode. FIGs. 11A and 11B corresponds to FIGs. 2A and 2B, respectively. In other words, the hand grip 21 at the time of the normal mode is configured as the bioelectrical impedance measurement apparatus for the right arm that is provided with two electrodes, i.e. the electrical-current electrode 1a and the voltage electrode 1b, similarly to the hand grip 1 in the first embodiment. However, as shown by dotted lines in the figure, the hand grip 21 of this embodiment is configured of at least four electrodes A to D arranged adjacent with each other, and furthermore, the voltage electrode 1b serving as the measurement electrode is configured by bringing these four electrodes A to D into mutual continuity.

FIGs. 11C and 11D are diagrams for explaining the configuration of the hand grip 21 at the time of the sebum caliper mode. In the hand grip 21 at the time of the sebum caliper mode, a portion corresponding to the voltage electrode 1b shown in FIGs. 11A and 11B is divided into four electrodes, i.e., an electrical-current electrode 21a+, an electrical-current electrode 21a-, a voltage electrode 21b+, and a voltage electrode 21b-. In other words, in the hand grip 21, the four electrodes functioning at the time of the sebum caliper mode are realized by bringing the four electrodes A to D, which configure a single electrode (the voltage electrode 1b) by being brought into the mutual continuity at the time of the normal mode, into mutual non-continuity. Furthermore, a portion corresponding to the electrical-current electrode 1a shown in FIGs. 11A and 11B is caused to lose its function as the electrode by bringing it into non-continuity with the electrical-current application part 9a, for example. By doing so, the hand grip 21 at the time of the sebum caliper mode can realize the function as the bioelectrical impedance measurement apparatus (a skinfold caliper) for local measurement that is provided with the four electrodes, i.e. the electrical-current electrode 21a+, the electrical-current electrode 21a-, the voltage electrode 21b+, and the voltage electrode 21b-.

In the above description, a method of bringing the above-described adjacent four electrodes A to D into continuity/non-continuity is not particularly limited, and in order to realize this configuration, for example, a switching circuit (not shown) for switching ON/OFF according to a control signal from a mode switching part 26, which will be described below, may be provided between the adjacent electrodes among the four electrodes A to D. In addition, points for switching the continuity/non-continuity of the four electrodes A to D may not necessarily be at between the electrodes A to D provided in the hand grip 21 as shown in the figure, and they may be at any points on the line between the respective electrodes of the electrodes A to D and the control part 20 for achieving the continuity.

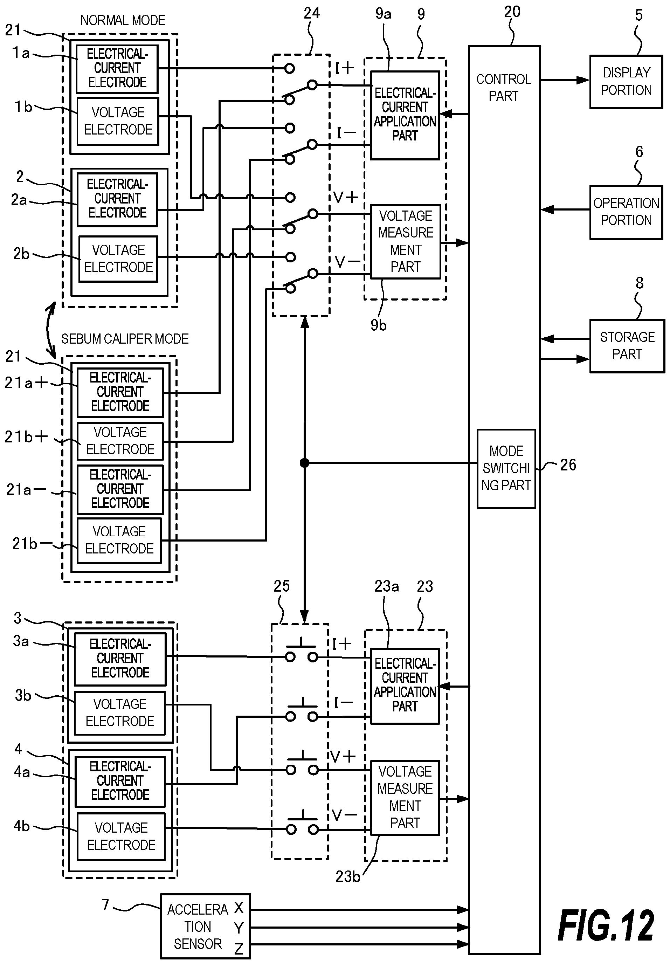

FIG. 12 is a block diagram showing a main functional configuration of the body composition analyzer 10 in this embodiment. This figure also shows the foot-side measurement portions 3, 4, and the hand grip 2 at the time of the normal mode, which are omitted in the first embodiment.

The body composition analyzer 10 in this embodiment is mainly provided with, as its functional configuration, in addition to the hand grip 2, the foot-side measurement portion 3, the foot-side measurement portion 4, the display portion 5, the operation portion 6, the acceleration sensor 7, the storage part 8, the bioelectrical impedance measurement part (the hand-side bioelectrical impedance measurement part) 9, and the control part 20: the hand grip 21; a foot-side bioelectrical impedance measurement part 23;, a hand-side mode switch 24; a foot-side mode switch 25; and a mode switching part 26.

The hand-side bioelectrical impedance measurement part 9 is configured of the electrical-current application part 9a having two output lines (I+, I-) for applying the electrical current and the voltage measurement part 9b having two input lines (V+, V-) for detecting the voltage. Respective connections (electrical connections) of the output lines (I+, I-) of the electrical-current application part 9a and the input lines (V+, V-) of the voltage measurement part 9b with the respective electrodes provided in the hand grip 21 and the hand grip 2 are switched in accordance with the respective measurement modes (the normal mode or the sebum caliper mode) via the hand-side mode switch 24.

The foot-side bioelectrical impedance measurement part 23 is configured of an electrical-current application part 23a having the two output lines (I+, I-) for applying the electrical current and a voltage measurement part 23b having the two input lines (V+, V-) for detecting the voltage. Respective connections of the output lines (I+, I-) of the electrical-current application part 23a and the input lines (V+, V-) of the voltage measurement part 23b with the respective electrodes provided in the foot-side measurement portion 3 and the foot-side measurement portion 4 are switched in accordance with the respective measurement modes (the normal mode or the sebum caliper mode) via the foot-side mode switch 25.

The mode switching part 26 switches the measurement mode of the body composition analyzer 10 on the basis of the angles of the hand grip 21 (at least one of the X-axis angle, the Y-axis angle, and the Z-axis angle) detected by the acceleration sensor 7. More specifically, the mode switching part 26 switches the measurement mode of the body composition analyzer 10 by controlling the hand-side mode switch 24 and the foot-side mode switch 25 on the basis of the angle of the hand grip 21 detected by the acceleration sensor 7. For example, when the mode switching part 26 detected that the user is maintaining a state in which the hand grip 21 is held horizontally with respect to the ground surface for the certain period of time on the basis of the detected angle of the hand grip 21, the mode switching part 26 controls the hand-side mode switch 24 and the foot-side mode switch 25 such that the measurement mode of the body composition analyzer 10 is switched to "the normal mode".

On the other hand, when the mode switching part 26 detected that the user is maintaining a state in which the hand grip 21 is held vertically with respect to the ground surface for the certain period of time on the basis of the detected angle of the hand grip 21, the mode switching part 26 controls the hand-side mode switch 24 and the foot-side mode switch 25 such that the measurement mode of the body composition analyzer 10 is switched to "the sebum caliper mode". In the above description, it may be possible to appropriately set which of the angle of the hand grip 21 and a period during which the angle is maintained serves as a trigger for the respective measurement modes.

The hand-side mode switch 24 switches the connections between the input/output lines of the hand-side bioelectrical impedance measurement part 9 and the respective electrodes provided in the hand grip 2 and 22 in accordance with a control signal (a mode switching signal) from the mode switching part 26.

In an example illustrated in this embodiment, when the measurement mode is "the sebum caliper mode", the hand-side mode switch 24 connects the output line I+ and the output line I- of the electrical-current application part 9a to the electrical-current electrode 21a+ and the electrical-current electrode 21a-, respectively, and the hand-side mode switch 24 connects the input line V+ and the input line V- of the voltage measurement part 9b to the voltage electrode 21b+ and the voltage electrode 21b-, respectively. By doing so, it is possible to set the measurement mode of the body composition analyzer 10 to the sebum caliper mode. The connections by the hand-side mode switch 24 shown in FIG. 12 are the connections at the time of "the sebum caliper mode".

On the other hand, when the measurement mode is "the normal mode", the hand-side mode switch 24 connects the output line I+ and the output line I- of the electrical-current application part 9a to the electrical-current electrode 1a and the electrical-current electrode 2a, respectively, and the hand-side mode switch 24 connects the input line V+ and the input line V- of the voltage measurement part 9b to the voltage electrode 1b and the voltage electrode 2b, respectively. By doing so, it is possible to set the measurement mode of the body composition analyzer 10 to the normal mode.

The foot-side mode switch 25 switches the connections between the input/output lines of the foot-side bioelectrical impedance measurement part 23 and the respective electrodes of the foot-side measurement portion 3 and 4 in accordance with the control signal (the mode switching signal) from the mode switching part 26.

In an example illustrated in this embodiment, when the measurement mode is "the sebum caliper mode", the foot-side mode switch 25 disconnects the input/output lines of the foot-side bioelectrical impedance measurement part 23 from the respective electrodes provided in the foot-side measurement portion 3 and 4. With such a configuration, it is possible to set the measurement mode of the body composition analyzer 10 to the sebum caliper mode. The connections by the foot-side mode switch 25 shown in FIG. 12 are the connections at the time of "the sebum caliper mode".

On the other hand, when the measurement mode is "the normal mode", the foot-side mode switch 25 connects the output line I+ and the output line I- of the electrical-current application part 23a to the electrical-current electrode 3a and the electrical-current electrode 4a, respectively, and the foot-side mode switch 25 connects the input line V+ and the input line V- of the voltage measurement part 23b to the voltage electrode 3b and the voltage electrode 4b, respectively. By doing to, it is possible to realize a four-electrode function provided in the foot-side of the eight-electrode type body composition analyzer 10.

When "the normal mode" of the body composition analyzer 10 is to be configured as the four-electrode type body composition analyzer using the hand grip 2 and 21, similarly to "the sebum caliper mode" described above, it suffices that the control part 20 is caused to lose the function of the foot-side measurement portions 3, 4 by disconnecting the input/output lines of the foot-side bioelectrical impedance measurement part 23 from the respective electrodes provided in the foot-side measurement portion 3 and 4. Alternatively, as the original configuration, the foot-side bioelectrical impedance measurement part 23, the foot-side mode switch 25, and the foot-side measurement portions 3, 4 may be deleted from the configuration of the body composition analyzer 10.

In the following, the details of a processing (a mode switching processing) for switching the measurement mode of the body composition analyzer 10 executed by the body composition analyzer 10 in this embodiment will be described. In this example, an example in which the measurement mode is switched between "the normal mode" and "the sebum caliper mode" by setting a mode of the body fat analyzer for measuring the body fat percentage of the user as "the normal mode" and a mode for locally measuring the sebum thickness of the user by using the hand grip 21 as "the sebum caliper mode". The mode switching processing, which will be described below, is realized on the basis of the control program stored in the storage part 8.

The mode switching processing, which will be described below, is started by assuming that the user is at least holding the hand grip 21.

In step S20, the mode switching part 26 acquires the angles of the hand grip 21 (at least one of the X-axis angle, the Y-axis angle, and the Z-axis angle) detected by the acceleration sensor 7 provided in the hand grip 21. At this time, in order to set the mode to the desired measurement mode, the user inclines the hand grip 21 to the angles corresponding to the respective modes. For example, in a case in which the angle corresponding to "the normal mode" is set as an angle condition α and the angle corresponding to "the sebum caliper mode" is set as an angle condition β, when the user is to select the normal mode, the user inclines the hand grip 21 for the certain period of time so as to satisfy the angle condition α.

As described above, the angle conditions α, β may be set appropriately. For example, the angle that is achieved when the longitudinal direction of the hand grip 21 is inclined horizontally with respect to the ground surface may be set as the angle condition α, and the angle that is achieved when the longitudinal direction of the hand grip 21 is inclined vertically with respect to the ground surface may be set as the angle condition β. In addition, a certain angle may be set as one of the angle conditions (for example, the angle condition α), and angles other than the angle condition α may be set as the angle condition β.

In step S21, the mode switching part 26 determines whether or not the angle condition α is maintained for the certain period of time. The certain period of time in this description may be set appropriately from the viewpoint of suitably reading the angle of the hand grip 21 intended by the user, and it may be three seconds for example. When it is determined that the angle condition α is maintained for the certain period of time, the processing in step S22 is executed. When it is determined that the angle condition α is not maintained for the certain period of time, the processing in step S24 is executed.

In step S22, the mode switching part 26 controls the hand-side mode switch 24 and the foot-side mode switch 25 to set the arrangements (the connections) of the electrodes of the hand grips 2 and 21 and the foot-side measurement portions 3 and 4 to the normal mode (see FIGs. 11A, 11B, and 12). At this time, the control part 20 may inform of the user that the measurement mode of the body composition analyzer 10 has been set to "the normal mode" via the display portion 5.

Then, in step S23, the control part 20 starts the measurement of the bioelectrical impedance of the user as the eight-electrode or four-electrode type body fat analyzer and terminates the mode switching processing. In the above, after the mode switching processing is finished, the above-described body composition measurement processing in the first embodiment is performed, or alternatively, the conventionally known body composition measurement processing is performed without performing the correction according to the detected value by the acceleration sensor 7 (the inclination angle) as described in the first embodiment.

On the other hand, in step S24, the mode switching part 26 determines whether or not the angle condition β is maintained for the certain period of time. When it is determined that the angle condition β is maintained for the certain period of time, the processing in step S25 is executed. When it is determined that the angle condition β is not maintained for the certain period of time, in order to acquire the angle of the hand grip 21 again, the processing in step S20 is executed. As described above, when the angles other than the angle compatible with the angle condition α are set as the angle condition β, step S24 may be omitted. In this case, when a negative determination (NO determination) is made in step S21, step S25 is executed subsequently.

In step S25, the mode switching part 26 controls the hand-side mode switch 24 and the foot-side mode switch 25 to set the arrangements (the connections) of the electrodes of the hand grips 2 and 21 and the foot-side measurement portions 3 and 4 to the sebum caliper mode (see FIGs. 11C, 11D, and FIG. 12). At this time, the control part 20 may inform of the user that the measurement mode of the body composition analyzer 10 has been set to "the sebum caliper mode" via the display portion 5.

Then, in step S26, the control part 20 starts the measurement of the bioelectrical impedance as the sebum caliper using the hand grip 21 provided with the four electrodes and terminates the mode switching processing. In the above, after the mode switching processing is finished, the control part 20 measures the local sebum thickness of the user by known methods. With the flow described above, the user can easily switch the two functions of the body composition analyzer 10 by a simple operation of changing the inclination of the hand grip 21.

Next, operational advantages of this embodiment will be described.

According to the body composition analyzer 10 in this embodiment, the hand grip 21 is configured so as to be capable of switching a first measurement mode (the normal mode) in which the pair of energization electrode (the electrical-current electrode 1a) and the measurement electrode (the voltage electrode 1b) are functioned and a second measurement mode (the sebum caliper mode) in which the two pairs of local energization electrode (the electrical-current electrode 21a+, 21a-) and the local measurement electrode (the voltage electrode 21b+, 21b-) are functioned, and the body composition analyzer 10 is further provided with the mode switching means (the mode switching part 26) that switches between the first measurement mode and the second measurement mode in accordance with the inclination of the hand grip 21 detected by the inclination detection means (the acceleration sensor 7). By doing so, it is possible to realize the body composition analyzer 10 that has both of the function of the four-electrode type or eight-electrode type conventional body composition analyzer compatible with the first measurement mode and the function (the second measurement mode) of the four-electrode type sebum caliper (an abdominal fat meter) compatible with the second measurement mode, and further, that is capable of switching these two functions.

As a result, because the user can utilize the respective functions by a single apparatus without obtaining two separate apparatuses respectively having the functions, it is advantageous in terms of a cost and an installation space of the apparatus. In addition, according to such a body composition analyzer 10, after the body fat percentage of a whole body has measured, it is possible to measure the local sebum thickness in a continuous operation, and therefore, it is advantageous for the user in that multilateral measurement of the body composition of himself/herself can be achieved by a simple operation. In addition, with such an advantage, the body composition analyzer 10 in this embodiment can improve a motivation of the user to measure the body composition of himself/herself in more detail.

In addition, according to the body composition analyzer 10 in this embodiment, one of the electrical-current electrode 1a and the voltage electrode 1b is configured of the at least four adjacent the electrodes A to D, and the mode switching means (the mode switching part 26) configures, when the hand grip 21 is set to the first measurement mode, a pair of the energization electrode (the electrical-current electrode 1a) and the measurement electrode (the voltage electrode 1b) in the hand grip 21 by bringing the at least four electrodes A to D into mutual continuity, and configures, when the hand grip 21 is set to the second measurement mode, two pairs of the local energization electrodes (the electrical-current electrode 21a+, 21a-) and the local measurement electrodes (the voltage electrode 21b+, 21b-) in the hand grip 21 by bringing the at least four electrodes A to D into mutual non-continuity so as to be electrically divided into four parts and by bringing the other of the energization electrode (the electrical-current electrode 1a) and the measurement electrode (the voltage electrode 1b) into non-continuity. By doing so, the electrode that is used for the function corresponding to the first measurement mode and the electrode that is used for the function corresponding to the second measurement mode can be at least partially shared, and therefore, it is possible to suppress a total number and the installation space of the electrodes required to realize the body composition analyzer 10 having the two functions described above.

Although the embodiments of the present invention have been described in the above, the above-mentioned embodiments merely illustrate a part of application examples of the present invention, and the technical scope of the present invention is not intended to be limited to the specific configurations of the above-described embodiments.

For example, the electrode part may not necessarily be the hand grip as described above by referring to FIG. 2, etc. It suffices that the electrode part has, upon the measurement of the bioelectrical impedance of the user, functions similar to those of the above-described hand grip 1, 2, and at the same time, has a shape that enable it to be fixed to the upper limb of the user. Specifically, the electrode part may be configured to have, for example, a clamping part having a clip-like shape so as to be fixable to the user by clamping the upper limb of the user (for example, the forearm part) by the clamping part.

For example, the inclination detection means is not limited to the acceleration sensor 7. Other sensors such as the gyro sensor, etc. may be used as the inclination detection means as long as the inclination of the hand grip 1 can be detected as the parameter indicative of the posture of the user during the measurement of the bioelectrical impedance. In addition, the inclination detection means is not limited to sensors, and the inclination of the hand grip may be detected by using a known mechanism, an image recognition, or the like.

In addition, the shape of the body composition analyzer 10 is not limited to those described by using the drawings, etc., and as long as the above-described functions are attained, it may be changed appropriately. For example, the shape of the hand grip 1, 2, 21 is not limited to those illustrated, and it may be changed appropriately by considering the ease of grasp, the stability of the posture of the user at the time of the measurement, and so forth.

In addition, the configuration of the body composition analyzer 10 is also not limited to those described by using the drawings, etc., and as long as the above-described functions are attained, it may be changed appropriately. For example, the arrangement of the respective electrodes provided in the hand grip 1, 2, 21 can be exchanged appropriately as long as the above-described respective functions operate suitably in accordance with the measurement mode. In addition, the electrodes A to D may not necessarily be the four electrodes that are adjacent with each other in a line, as described by using FIGs. 11A to 11D. In the above-described sebum caliper mode, the electrical-current electrode 1a or the voltage electrode 1b of the hand grip 21 may be configured of five or more electrodes as long as they can be divided into adjacent four electrodes. For example, when the electrical-current electrode 1a or the voltage electrode 1b is configured of five electrodes, the electrical-current electrode 1a or the voltage electrode 1b may be configured so as to be capable of being divided into the four electrodes in mutual non-continuity by bringing two adjacent electrodes into continuity and by bringing these two electrode and the other three electrodes into mutual non-continuity.

In addition, the directions (the X axis direction, the Y axis direction, and the Z axis direction) detected by the acceleration sensor 7 provided in the above-described hand grips 1, 2, 21 are examples, and they can be changed appropriately. In addition, the abnormal posture of the user detected in accordance with the respective axial directions described with reference to FIGs. 8 to 10 are examples, and they are not limited to those illustrated. The axial directions detected by the acceleration sensor 7 in accordance with the inclinations of the hand grips 1, 2, 21 and the abnormal posture detected by the change in the axial directions may be adjusted or changed appropriately.

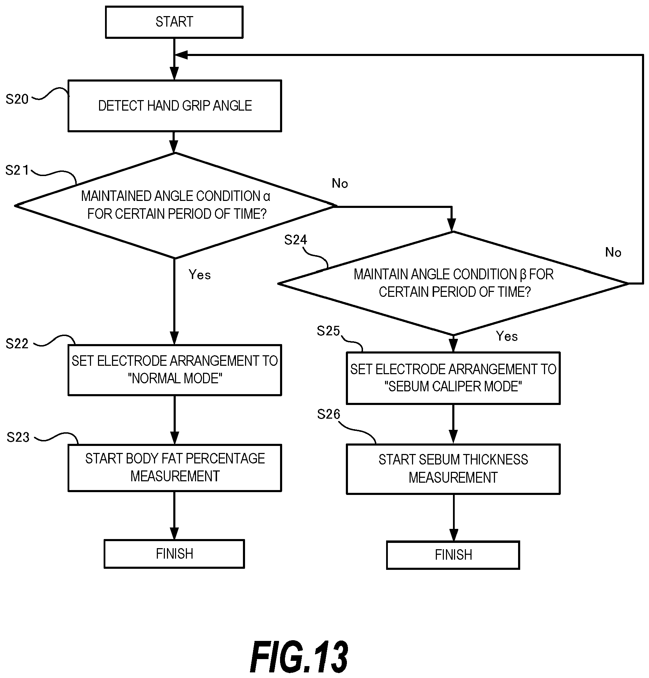

In addition, the flowcharts shown in FIGs. 6 and 13 may not necessarily include all of the illustrated steps, and may not be executed in the order illustrated. In addition, as long as the above-described functions are not lost, the flowcharts may include other steps that are not illustrated. For example, as a measurement start condition for the body composition measurement processing shown in FIG. 6, a step of determining whether or not the normal posture (the reference value) is detected may be added. By adding such a step, in the body composition measurement processing shown in FIG. 6, an action of taking the normal posture at least once by the user grasping the hand grips 1 and 2 can be set as the measurement start condition.

In addition, the mode switching processing described above in the second embodiment is not limited to the mode described in FIG. 13. For example, the mode switching processing may be configured such that, when the inclination angle falling outside the predetermined range is detected (for example, see the NO determination in steps S12, S14, and S16) during the measurement in the normal mode as shown in FIG. 6, the mode is switched to the sebum caliper mode, and as the measurement in the sebum caliper mode is finished, the mode is switched back to the normal mode again to start the measurement of the body fat percentage again. In this case, when the mode is switched to the sebum caliper mode and when the mode is switched back to the normal mode again, it is preferable to inform the user of the switching of the operating mode.

In addition, the above-described mode switching processing may not necessarily be triggered by the inclination angle detected by the acceleration sensor 7 for the switching of the mode. For example, the mode switching processing may be configured so as to be switched in accordance with the operation of the user acquired via the operation portion 6. In addition, the mode switching processing may be configured such that the operation modes are switched as a series of measurement processing executed in the respective operation modes without requiring a predetermined switching operation by the user, by for example, automatically switching the mode to the sebum caliper mode after the measurement in the normal mode is finished, or by automatically switching the mode to the normal mode after the measurement in the sebum caliper mode is finished.

In the above description, the terms indicating the directions such as the horizontal (the parallel) direction, the vertical direction, and so forth, which are used especially for the description of the hand grip 1, 2, 21 or the description of the axial direction to be detected by the acceleration sensor 7, are not necessarily intended to indicate the accurate directions in the strict sense. The directions stated in this description may include some deviations within the allowable range from the viewpoint of the measurement accuracy of the body composition measured by the body composition analyzer 10.

Although the embodiments of the present invention have been described in the above, the above-mentioned embodiments merely illustrate a part of application examples of the present invention, and the technical scope of the present invention is not intended to be limited to the specific configurations of the above-described embodiments.

The present application claims priority to Japanese Patent Application No. 2019-68650, filed in the Japan Patent Office on March 29, 2019. The contents of this application are incorporated herein by reference in their entirety.

### Reference Signs List

1, 2, 21 hand grip (electrode part)
1a, 2a electrical-current electrode (energization electrode)
1b, 2b voltage electrode (measurement electrode)
7 acceleration sensor (inclination detection means)
10 body composition analyzer (biological data measurement apparatus)
20 control part (body composition calculation means, abnormal posture determination means, abnormal-posture-degree calculation means)
21a+, 21a- electrical-current electrode (local energization electrode)
21b+, 21b- voltage electrode (local measurement electrode)
22 biological information correction part (correction means)
26 mode switching part (mode switching means)
27 biological information calculation part (biological-information measurement means)
S10 (inclination detection step)
S13, S15, S17 (correction step)
S18 (body composition calculation step)
S19 (informing step)

## Claims

1. A biological data measurement apparatus comprising:
an electrode part provided with an energization electrode and a measurement electrode, the electrode part being fixable to an upper limb of a user, and the energization electrode and the measurement electrode being arranged so as to be separated from each other;
biological-information measurement means configured to measure biological information of the user by using the electrode part;
inclination detection means configured to detect an inclination of the electrode part; and
correction means configured to correct a measurement result obtained by the biological-information measurement means in accordance with the inclination of the electrode part detected by the inclination detection means.

2. The biological data measurement apparatus according to claim 1, wherein
the inclination detection means is an acceleration sensor provided in the electrode part.

3. The biological data measurement apparatus according to claim 1 or 2, wherein
the correction means is configured to correct the measurement result when it is determined that the inclination of the electrode part does not fall within a predetermined inclination range.

4. The biological data measurement apparatus according to claim 3, wherein
the predetermined inclination range is set based on an inclination of the electrode part at the time when the user, to which the electrode part is fixed, is maintaining a posture recommended for measuring the biological information.

5. The biological data measurement apparatus according to any one of claims 1 to 4, wherein
the measurement result contains a bioelectrical impedance of the user or a body composition of the user.

6. The biological data measurement apparatus according to claim 5, further comprising
when the measurement result is the bioelectrical impedance of the user,
body composition calculation means configured to calculate the body composition of the user based on the bioelectrical impedance corrected by the correction means.

7. The biological data measurement apparatus according to claim 5 or 6, wherein
the correction means is configured to calculate a difference between the inclination of the electrode part at the time when the user, to which the electrode part is fixed, is maintaining the posture recommended for measurement of the biological information and the inclination of the electrode part detected by the inclination detection means, the correction means being configured to perform correction such that the bioelectrical impedance takes smaller value as the difference calculated is increased.

8. The biological data measurement apparatus according to any one of claims 1 to 7, wherein
the electrode part is configured so as to be capable of being grasped by a hand of the user, the electrode part being configured so as to be capable of switching a first measurement mode and a second measurement mode, the first measurement mode being configured to allow a pair of the energization electrode and the measurement electrode to function, and the second measurement mode being configured to allow two pairs of local energization electrodes and local measurement electrodes to function, and
the biological data measurement apparatus further comprising mode switching means configured to switch between the first measurement mode and the second measurement mode in accordance with the inclination of the electrode part detected by the inclination detection means.

9. The biological data measurement apparatus according to claim 8, wherein
one of the energization electrode and the measurement electrode is configured of at least four adjacent electrodes,
the mode switching means configures:
when the electrode part is set to the first measurement mode, a pair of the energization electrode and the measurement electrode in the electrode part by bringing the at least four electrodes into mutual continuity; and
when the electrode part is set to the second measurement mode, two pairs of the local energization electrodes and the local measurement electrodes in the electrode part by bringing the at least four electrodes into mutual non-continuity so as to be electrically divided into four parts and by bringing other of the energization electrode and the measurement electrode into non-continuity.

10. A program configured to cause a computer to execute:
a biological information measurement step of measuring biological information of a user by using an electrode part, the electrode part being fixable to an upper limb of the user, and the electrode part having an energization electrode and a measurement electrode arranged so as to be separated from each other;
an inclination detection step of detecting an inclination of the electrode part; and
a correction step of correcting a measurement result obtained in the biological information measurement step, the measurement result being corrected in accordance with the inclination of the electrode part detected in the inclination detection step.

11. A biological data measurement method comprising:
a biological information measurement step of measuring biological information of a user by using an electrode part, the electrode part being fixable to an upper limb of the user, and the electrode part having an energization electrode and a measurement electrode arranged so as to be separated from each other;
an inclination detection step of detecting an inclination of the electrode part; and
a correction step of correcting a measurement result obtained in the biological information measurement step, the measurement result being corrected in accordance with the inclination of the electrode part detected in the inclination detection step.
